Europäisches Patentamt

European Patent Office

Office européen des brevets

(19)

(11) Publication number: **0 034 049**
**B1**

(12)

# EUROPEAN PATENT SPECIFICATION

(45) Date of publication of patent specification: **02.01.85**

(21) Application number: **81300494.2**

(22) Date of filing: **05.02.81**

(51) Int. Cl.⁴: **B 01 L 3/00,** G 01 N 1/28,
G 01 N 33/52

(54) **Test device for analysis of a plurality of analytes.**

(30) Priority: **06.02.80 US 118840**

(43) Date of publication of application:
**19.08.81 Bulletin 81/33**

(45) Publication of the grant of the patent:
**02.01.85 Bulletin 85/01**

(84) Designated Contracting States:
**DE FR GB**

(56) References cited:
**EP-A-0 014 797**
**DE-A-2 934 760**
**DE-A-2 949 747**
**FR-A-1 598 197**
**FR-A-2 325 920**
**US-A-3 690 836**
**US-A-3 715 192**
**US-A-3 876 377**
**US-A-3 992 158**

(73) Proprietor: **EASTMAN KODAK COMPANY**
**343 State Street**
**Rochester New York 14650 (US)**

(72) Inventor: **Columbus, Richard Lewis**
**Kodak Park**
**Rochester New York (US)**

(74) Representative: **Pepper, John Herbert et al**
**KODAK LIMITED Patent Department P.O. Box**
**114 190 High Holborn**
**London WC1V 7EA (GB)**

Courier Press, Leamington Spa, England.

## Description

The present invention relates to a device useful in the clinical analysis of analytes of a liquid.

US—A—3,992,158, discloses a new approach to chemical analysis in which a dry test element contains all the necessary reagents for an extremely accurate quantitative assay on a single drop of test liquid, such as blood serum. The test element is provided with one or more reagent layers and a spreading layer; the spreading layer is preferably isotropically porous to insure that uniform concentrations of the dissolved or dispersed substances of the test liquid arrive at the reagent layers for the necessary chemical reactions. The result is a uniform generation of a detectable response predictably related to the concentration of the analyte under study.

The above-described test elements provided a marked improvement in the state of the art, since they eliminated the plumbing required in apparatus using "wet" techniques and improved the accuracy of the test results. It was the practice to provide a separate test element for each assay, for example one element for the assay of glucose and another for the assay of blood urea nitrogen (BUN). It was recognized, however, that there would be significant advantages in a test element of this type which could be used for a number of different assays.

It is known to provide a single test device for performing a plurality of assays. However, devices developed heretofore have not been generally successful, since reactions from the different assays tend to interfere with each other. One such device is disclosed in US—A—3,715,192, in which a dip-and-read element is described as having two different reagent layers spaced from each other to define a path through which the test liquid flows. Reagent from one layer is free to flow into the other layer along the common flow path, and thus, there is potential for cross-contamination. Although a certain amount of cross-contamination can be ignored in qualitative devices, it cannot be ignored when making exact, quantitative measurements.

In US—A—3,690,836, a multiple test element is disclosed which incorporates a plurality of test devices on a single support. Each of the test devices comprises superposed plastic sheets sealed together to define a capillary reaction chamber, one of the sheets having an aperture therein to provide access to the chamber, and an absorbent sheet in the chamber which is adapted to contain a reagent. The disadvantage of elements of this type is that a separate drop of test liquid must be supplied to each of the test devices; consequently, separate metering operations are required for each assay.

It is an object of the present invention to overcome the aforementioned problems in the prior art by providing a multi-analyte device for performing a plurality of assays on a single drop of test liquid without interference between the reactions of the respective assays.

In accordance with the present invention there is provided a test device for the analysis of a plurality of analytes in a liquid, said device comprising a support member and a cover member, said cover and support members having opposed surfaces defining a liquid transport zone in which the surfaces are spaced apart a distance effective to induce capillary flow of said liquid, said device having access means in one of said cover and support members for receiving and transporting a quantity of the liquid into said zone, said zone having therein a plurality of reagent compositions for the generation of detectable responses indicative of analytes, said support member being provided in the vicinity of said access means with a boss, said boss and said cover member being configured to create a temporary energy barrier to liquid flow beyond the vicinity of said access means, said boss having a surface spaced a capillary distance from the surface of the cover member, a plurality of individual test elements being disposed in said zone on said support member at locations spaced from each other and from said access means and having surfaces spaced a capillary distance from the surface of the cover member, one of said elements comprising one of said reagent compositions and another of said elements comprising another of said reagent compositions, and said surface of the boss and the opposed portion of said cover member being raised relative to, respectively, said surfaces of the test elements and portions of said cover member directly opposite to said test elements, and comprising control means for confining liquid flow to a plurality of predetermined flow paths each of which extends, completely independently of the other paths, from the vicinity of said access means to only one of said test elements whereby the analytes in said quantity of liquid are detectable by the respective test elements without cross-contamination between the test elements.

The present invention will now be described, by way of example, with reference to the accompanying drawings in which:

Fig. 1 is a plan view of a test device in accordance with the invention;

Fig. 2 is a sectional view taken generally along the plane of the line II—II in Fig. 1; and

Fig. 3 is a plan view similar to that of Fig. 1, but illustrating the liquid flow paths to each test element.

The device and the method of this invention are described, in connection with certain preferred embodiments, as a device and a method for analyzing blood serum. In addition, the invention can be applied in the radiometric detection of analytes of other liquids, for example, industrial liquids.

European Patent Application No.

79302338.3 (EP—A—0,014,797), published September 3, 1980, entitled "Liquid Transport Device, Method of Transporting A Liquid, Method of Analyzing A Liquid, and Test Element for Use in Analysis of a Liquid," describes generically the use of opposed transport surfaces to distribute a liquid drop by capillary action to a plurality of test areas. The present invention is an improvement on the invention disclosed in EP—A—0,014,797.

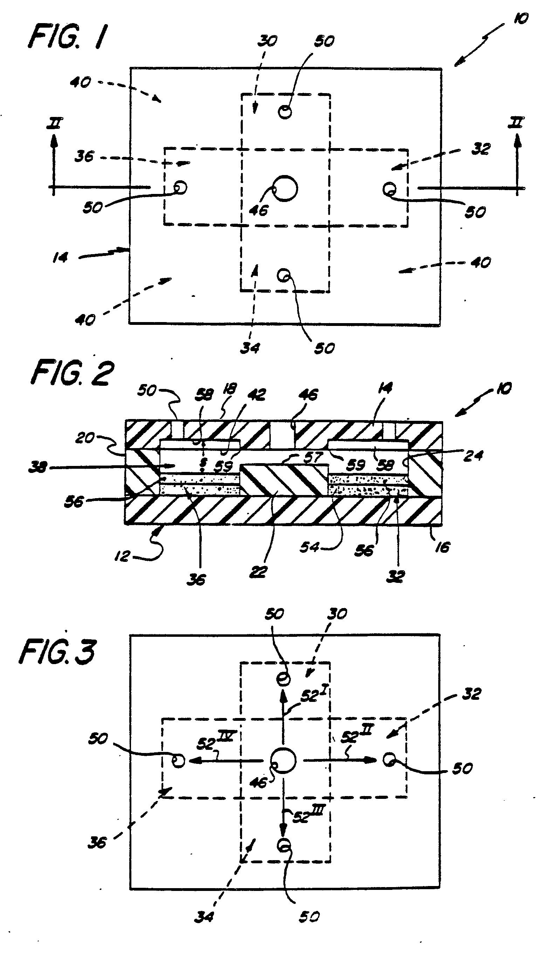

In Figs. 1 and 2, there is shown a device 10 constructed in accordance with one embodiment of the invention. Device 10 comprises a support member 12, preferably transparent, and a cover member 14 which have exterior surfaces 16 and 18, respectively. Members 12 and 14 are sealed at their peripheries to an intermediate member 20 (Fig. 2), the sealing being effected for example by an appropriate adhesive or by ultrasonic welding. It will be apparent that members 12, 14 and 20 could be integrally formed by conventional techniques. Except for apertures 46, 50, discussed hereinafter, the space enclosed by members 12, 14 and 20 is preferably airtight.

At the centre of the interior surface of support member 12 is disposed a boss 22 which, together with the interior wall 24 of member 20, defines a plurality of cavities extending radially from the boss. These cavities are filled with a plurality of individual test elements 30, 32, 34 and 36 (Fig. 1) each of which contains all the reagents necessary for the detection of a different one of the several analytes expected to be present in the test liquid. Intermediate member 20 includes a spacer 40 (Fig. 1) at each of the corners of device 10 to fill portions of the cavities not filled by the test elements.

The top surfaces of test elements 30, 32, 34 and 36, as well as that of the boss 22, are spaced a capillary distance from an interior surface 42 of cover member 14 (Fig. 2) that is effective to induce capillary flow of liquid introduced between the cover member 14 and either the boss 22 or the test elements, and to thus create a liquid transport zone 38. Zone 38 is bounded in the vertical direction, as viewed in Fig. 2, by wall 24 and spacers 40 of intermediate member 20. Preferably said capillary distance is between about 25 microns and about 500 microns. To permit the introduction of the test liquid, an access aperture 46 is formed in cover member 14, which functions as a receiving zone. Alternatively, the access aperture can be provided in the centre portion of member 12 through boss 22. The size and shape of the access aperture 46 is such as to accommodate a drop of test liquid while at the same time ensuring that the drop will contact both surface 42 and the boss 22. Because of the capillary spacing, such contact immediately causes the drop to spread under the influence of capillary attraction. Although the shape of the wall defining access aperture 46 is not critical, and can be cylindrical as shown, cross-sectional shapes such as a regular hexagon (not shown) provide interior corners that act to centre the drop within the aperture. To prevent cross-contamination of the reagents from the test elements, aperture 46 is not located immediately adjacent any test element.

For a 10 $\mu$l sized drop, a convenient size of such a hexagonally shaped access aperture is one in which the outside diameter is about 0.26 cm. The corners of the hexagon should be as sharply defined as possible, and in no case have a radius of curvature greater than about 0.4 mm.

As the liquid advances between surface 42 and the top surfaces of boss 22 and the test elements, the trapped air must exit. To provide a vent means for the trapped air, vent apertures 50 are formed in cover member 14 almost at the edge of zone 38 as defined by intermediate member 20. Each test element 30, 32, 34 and 36 has a vent aperture 50 disposed above it.

Liquid flow in zone 38 can occur only in the direction in which the trapped air is being expelled. Thus, in accordance with one aspect of the invention, a control means, which includes apertures 50 and intermediate member 20, confines the liquid to flow along a plurality of predetermined controlled flow paths (Fig. 3) such that each of the test elements 30, 32, 34 and 36 is operatively disposed in alignment with only one of those paths. The paths are identified in Fig. 3 as $52^I$, $52^{II}$, $52^{III}$, and $52^{IV}$, respectively, leading from aperture 46 to the vent aperture 50 associated with a particular test element. It is this construction that prevents significant contamination of one test element by a reagent of another test element, inasmuch as flow proceeds to test elements 30, 32, 34 and 36, rather than between them.

In accordance with another aspect of the invention, each of the test elements 30, 32, 34 and 36 comprises one or more layers that are more or less absorbent of the liquid under test. Such absorbency further prevents cross-flow between test elements which would create contamination. That is, as the sample liquid is absorbed, connective paths are removed between test elements that might otherwise remain after overall flow has ceased. Diffusion through quiescent liquid in the zone 38 is thus prevented.

Preferably each test element 30, 32, 34, and 36 comprises one or more reagent layers (Fig. 2) having a variety of binder compositions. For example, gelatin, cellulose acetate butyrate, polyvinyl alcohol, agarose and the like can be used, the degree of hydrophilicity of a layer 54 depending upon the material selected.

Additional layers, such as a layer 56, can be disposed above layer 54 to provide a variety of chemistries or functions. For example, these can provide, either in layer 56 alone or together with layer 54, a reagent composition. Filtering, registration and mordanting functions can be provided also by such additional layers, as described in US—A—4,042,335. Thus, layer 56 can comprise a reagent, such as an enzyme, and a binder.

As used herein, "reagent" in "reagent composition" means a material that is capable of interaction with an analyte, a precursor of an analyte, a decomposition product of an analyte, or an intermediate. Thus, one of the reagents can be a preformed, radiometrically detectable species that is caused by the analyte of choice to move out of a radiometrically opaque portion or layer of the element, such as layer 56, into a radiometrically transparent portion or layer, such as a registration layer which can be layer 54.

The noted interaction between the reagents of the reagent composition and the analyte is therefore meant to refer to chemical reaction, catalytic activity as in the formation of an enzyme-substrate complex, or any other form of chemical or physical interaction, including physical displacement, that can produce ultimately a detectable response in the test element. The assay of the element is designed to produce a response signal that is predictably related to the amount of analyte that is present.

The preferred device is designed for a radiometric detection of the response, that is, by impinging electromagnetic energy on the test elements. The response is then measured, preferably radiometrically. As is well known, radiometric detection includes both colorimetric and fluorimetric detection, depending upon the indicator reagent selected for the assay.

Alternatively, the device of the invention can be used with test elements having any kind of detectable response, the device being suitably modified, if necessary, to permit such alternate form of detection.

Each of the test elements 30, 32, 34 and 36 can test for a different analyte. Preferably, the assays are all oxygen-independent, as the flow of blood or blood serum into zone 38 tends to seal off the elements from any additional oxygen. Typical analytes which can be tested include BUN, total protein, bilirubin and the like. The necessary reagents and binder or vehicle compositions for, e.g. layers 54 and 56 of the test elements for BUN, total protein, and bilirubin, can be those described, respectively, in US—A—4,066,403; 4,132,528; and 4,069,016 or 4,069,017. Thus, test element 30 can be designed to test for BUN, element 32 to test for total protein, element 34 to test for bilirubin, and element 36 to test for a fourth analyte. Alternatively, element 36 can be a duplicate of any of elements 30, 32 or 34 to provide a confirming reading, or it can be a blank to permit standardization of the analyzer.

As is apparent from the preceding, the quantity of liquid preferably is added to aperture 46 and zone 38 in the form of a drop. Alternatively, other forms of liquid introduction can be used. For example, a pipette tip could be inserted into aperture 46 to dispense the desired quantity of liquid into zone 38.

Quantitative detection of the response produced in each test element by the analyte is preferably made, after a suitable development period, by scanning the device 10 through member 12 with a photometer or fluorimeter. Either a reflective or a transmissive mode can be used, depending upon the presence or absence of an opacifier in layer 56. A variety of such instruments can be used, for example the radiometer disclosed in US—A—4,152,390, or the photometer described in US—A—4,119,381. Of course, such instruments would be adapted to separately read each test element of device 10, such as through the use of filters and means to index the scanning beam to each test element. After detection, the device 10 is discarded.

Preferred materials for members 12, 14 and 20 are non-fibrous plastics materials that are substantially impervious to aqueous liquids. Examples include acetates, polystyrene, polyethylene, ABS plastic and polycarbonate.

Any other radial design can be used, besides that of Fig. 1, to provide, for example, 3, 5, 6 or n-test elements that are preferably equidistant from access aperture 46.

As an additional alternative, test elements 30, 32, 34 and 36 can be located on cover member 14 instead of member 12, or on both of said members, in suitably formed cavities therein.

A smooth, non-directional boss surface could result in exclusive flow to only one side, and one test element, of the test device. Therefore, to ensure that incoming liquid first completely wets all of the surface of boss 22, and then proceeds to all of the test elements, the following features are provided. Surface 57 of boss 22 is raised above the exterior surface of layers 56. In addition, surface 42 is recessed slightly at portions 58 which are disposed directly opposite to elements 32, 36, etc. Distance "s" between the recessed portions of surface 42 and the exterior surface of layer 56 is a capillary spacing, although larger than the spacing existing over boss 22. This embodiment is effective because the first portion of the liquid that enters zone 38 "sees" a temporary energy barrier at the edges of surface 57 and edges 59 in surface 42 created by the portions 58. The meniscus prefers to fill the zone directly above boss surface 57, rather than proceed into the wider spacing denoted as "s". After the zone directly above surface 57 is filled, however, the pressure of the incoming liquid overcomes the temporary energy barrier, and the liquid proceeds to fill the zone above all the test elements.

The interior surface 42 of the cover member 14 need not be smooth, as shown in Fig. 2. In accordance with another aspect of the invention, control of the liquid flow paths can also be obtained by portions of one or both opposed surfaces having a plurality of exposed grooves.

## Claims

1. A test device for the analysis of a plurality of analytes in a liquid, said device comprising a support member (12) and a cover member (14), said cover (14) and support (12) members having opposed surfaces defining a liquid transport zone (38) in which the surfaces are spaced apart a distance effective to induce capillary flow of said liquid, said device having access means (46) in one of said cover and support members (14, 12) for receiving and transporting a quantity of the liquid into said zone (38), said zone (38) having therein a plurality of reagent compositions for the generation of detectable responses indicative of analytes, said support member (12) being provided in the vicinity of said access means (46) with a boss (22), said boss (22) and said cover member (14) being configured to create a temporary energy barrier to liquid flow beyond the vicinity of said access means (46), said boss (22) having a surface (57) spaced a capillary distance from the surface (42) of the cover member (14), a plurality of individual test elements (30—36) being disposed in said zone (38) on said support member (12) at locations spaced from each other and from said access means (46) and having surfaces spaced a capillary distance from the surface (42) of the cover member (14), one of said elements (30—36) comprising one of said reagent compositions and another of said elements (30—36) comprising another of said reagent compositions, and said surface (57) of the boss (22) and the opposed portion of said cover member (14) being raised relative to, respectively, said surfaces of the test elements (30—36), and portions (58) of said cover member (14) directly opposite to said test elements (30—36), and comprising control means (20, 50) for confining liquid flow to a plurality of predetermined flow paths (52$^I$—52$^{IV}$) each of which extends, completely independently of the other paths, from the vicinity of said access means (46) to only one of said test elements (30—36) whereby the analytes in said quantity of liquid are detectable by the respective test elements (30—36) without cross-contamination between the test elements (30—36).

2. A device according to claim 1, wherein said control means includes a vent means (50) associated with each of said test elements (30—36) for venting air from said zone ahead of liquid flowing to the test element (30—36).

3. A device according to claim 1 or 2, wherein said test elements (30—36) each comprises an absorbent layer (54, 56) containing at least one reagent.

4. A device according to claim 1, 2 or 3, wherein each of said test elements (30—36) includes all the reagents necessary for a radiometric assay of a particular analyte.

5. A device according to claim 3, wherein said one element (30—36) includes the reagents necessary for the detection of total protein and said other element (30—36) includes the reagents necessary for the detection of blood urea nitrogen.

6. A device according to any one of claims 1 to 5, wherein said test elements (30—36) are equidistant from said access means (46).

7. A device according to any one of claims 1 to 6, wherein said control means includes a plurality of exposed grooves in at least one of said opposed surfaces.

8. A device according to any one of the preceding claims wherein one of said members (12, 14) is transparent.

## Revendications

1. Dispositif d'essai pour l'analyse d'analytes dans un liquide, qui comprend un élément support (12) et un élément de couverture (14), qui présentent des surfaces opposées définissant une zone de transfert de liquide (38) où les surfaces sont distantes d'une valeur apte à induire un écoulement capillaire de ce liquide, des moyens d'accès (46) dans l'un de ces éléments de couverture et support (14, 12) pour recevoir et transférer une quantité du liquide dans cette zone (38) ou se trouve une série de compositions réactives pour générer des réponses détectables indicatives des analytes, cet élément support (12) étant muni dans le voisinage de ces moyens d'accès (46) d'un renflement (22), ce renflement (22) et cet élément de couverture (14) étant configurés pour créer une barrière d'énergie temporaire à l'écoulement liquide au-delà du voisinage de ces moyens d'accès (46), ce renflement (22) présentant une surface (57) séparée d'une distance capillaire de la surface (42) de l'élément de couverture (14), une collection d'éléments d'essai indépendants (30—36) étant disposés dans cette zone (38) sur cet élément support (12) à des endroits séparés les uns des autres et de ces moyens d'accès (46) et présentant des surfaces séparées d'une distance capillaire de la surface (42) de l'élément de couverture (14), l'un de ces éléments (30—36) comprenant une de ces compositions reactives et un autre de ces éléments (30—36) comprenant une autre de ces compositions réactives et cette surface (57) du renflement (22) et les portions opposées de cet élément de couverture (14) étant en saillie relativement à, respectivement, ces surfaces des éléments d'essai (30—36) et portions (58) de cet élément de couverture (14) directement en vis à vis de ces éléments d'essai (30—36), et comprenant des moyens de régulation (20, 50) pour confiner un écoulement liquide vers des trajectoires d'écoulement prédéterminées multiples (52$^I$—52$^{IV}$) qui chacune s'étend, d'une manière complètement indépendante des autes trajectoires, du voisinage de ces moyens d'accès (46) à seulement l'un de ces éléments d'essai (30—36) de manière que les analytes de cette quantité de liquide soient détectables par les éléments d'essai respectifs (30—36) sans contamination mutuelle entre les éléments d'essai (30—36).

2. Dispositif conforme à la revendication 1 où ces moyens de régulation comprennent des évents (50) associés à chacun de ces éléments d'essai (30—36) pour évacuer l'air de ces zones en avant du liquide s'écoulant vers l'élément d'essai (30—36).

3. Dispositif conforme à la revendication 1 ou 2 où ces éléments d'essai (30—36) comprennent chacun une couche absorbante (54, 56) contenant au moins un réactif.

4. Dispositif conforme à la revendication 1, 2 ou 3 où chacun de ces éléments d'essai (30—36) comprend tous les réactifs nécessaires pour une analyse radiométrique d'un analyte particulier.

5. Dispositif conforme à la revendication 3 où cet élément (30—36) comprend les réactifs nécessaires pour le détection des protéines globales et cet autre élément d'essai (30—36) comprend les réactifs nécessaires à la détection de l'azote de l'urée du sang.

6. Dispositif conforme à l'une quelconque des revendications 1 à 5 où ces éléments d'essai (30—36) sont équidistants de ces moyens d'accès (46).

7. Dispositif conforme à l'une quelconque des revendications 1 à 6 où ces moyens de régulation comprennent une série de sillons apparents dans l'une au moins de ces surfaces en vis à vis.

8. Dispositif conforme à l'une quelconque des revendications précédentes où l'un de ses éléments (12, 14) est transparent.

**Patentansprüche**

1. Für die Analyse einer Vielzahl von Analyten in einer Flüssigkeit vorgesehene Testvorrichtung mit einem Trägerelement (12) und einem Abdeckelement (14), deren einander gegenüberliegende Flächen eine Flüssigkeitstransportzone (38) begrenzen, in der diese Flächen in einem solchen Abstand zueinander angeordnet sind, daß dadurch eine Kapillarströmung der Flüssigkeit erzeugt wird, ferner mit einer Einlaßeinrichtung (46), die entweder in dem Abdeck- oder dem Trägerelement (14, 12) vorgesehen ist und durch die eine Flüssigkeitsmenge aufgenommen und in die Transportzone (38) transportiert wird, die eine Vielzahl von Reagenzien enthält, die nachweisbare, für Analyten repräsentative Reaktionen zeigen, wobei das Trägerelement (12) benachbart der Einlaßeinrichtung (46) mit einem Vorsprung (22) versehen ist und der Vorsprung (22) und das Abdeckelement (14) so ausgebildet sind, daß sie für die Flüssigkeitsströmung außerhalb des Umgebungsbereichs der Einlaßeinrichtung (46) vorübergehend eine Energieschwelle bilden, wobei der Vorsprung (22) eine Fläche (57) besitzt, die in einem kapillaren Abstand von der Fläche (42) des Abdeckelements (14) angeordnet ist, wobei eine Vielzahl einzelner Testelemente (30—36) in der Transportzone (38) auf dem Trägerelement (12) an im Abstand voneinander und von der Einlaßeinrichtung (46) befindlichen Orten angeordnet sind und Flächen aufweisen, die sich in einem kapillaren Abstand von der Fläche (42) des Abdeckelements (14) befinden, und wobei eines der Testelemente (30—36) jeweils ein Reagens und ein anderes der Testelemente (30—36) ein anderes Reagens aufweist und die Fläche (57) des Vorsprungs (22) und der gegenüberliegende Abschnitt des Abdeckelements (14) in Bezug auf die Flächen der Testelemente (30—36) bzw. die den Testelementen (30—36) direkt gegenüberliegenden Abschnitte (58) des Abdeckelements (14) erhöht sind, und schließlich mit Steuermitteln (20, 50), durch welche die Flüssigkeitsströmung auf eine Vielzahl von vorbestimmten Strömungsbahnen ($52^I$—$52^{IV}$) beschränkt ist, von denen jede völlig unabhängig von den anderen Bahnen aus der Umgebung der Einlaßeinrichtung (46) zu weils nur einem der Testelemente (30—36) hin verläuft, wodurch die Analyten in der Flüssigkeitsmenge mittels des entsprechenden Testelements (30—36) nachweisbar sind, ohne daß zwischen den Testelementen (30—36) eine gegenseitige Verunreinigung auftreten kann.

2. Vorrichtung nach Anspruch 1, worin die Steuermittel eine jeweils den Testelementen (30—36) zugeordnete Entlüftungseinrichtung (50) aufweisen, durch die die Transportzone jeweils vor der zu dem Testelement (30—36) strömenden Flüssigkeit entlüftet wird.

3. Vorrichtung nach Anspruch 1 oder 2, worin jedes der Testelemente (30—36) eine mindestens ein Reagens enthaltende absorbierende Schicht (54, 56) aufweist.

4. Vorrichtung nach Anspruch 1, 2 oder 3, worin jedes der Testelemente (30—36) alle die für eine radiometrische Analyse eines bestimmten Analyten erforderlichen Reagenzien enthält.

5. Vorrichtung nach Anspruch 3, worin jeweils ein Testelement (30—36) die für die Bestimmung von Gesamtprotein und jeweils ein anderes Testelement (30—36) die für die Bestimmung von Blutharnstoff-Stickstoff erforderlichen Reagenzien enthält.

6. Vorrichtung nach einem der Ansprüche 1 bis 5, worin die Testelemente (30—36) alle gleich weit von der Einlaßeinrichtung (46) entfernt sind.

7. Vorrichtung nach einem der Ansprüche 1 bis 6, worin die Steuermittel eine Vielzahl offener Rillen in mindestens einer der einander gegenüberliegenden Flächen aufweisen.

8. Vorrichtung nach einem der vorhergehenden Ansprüche, worin eines der Elemente (12, 14) transparent ist.

FIG. 1

FIG. 2

FIG.3